# EUROPEAN PATENT SPECIFICATION

(11) **EP 3 801 274 B1**
(45) Date of publication and mention of the grant of the patent: **13.12.2023**
(21) Application number: 19726073.0
(22) Date of filing: 14.05.2019
(51) Int. Cl.: A61B 7/04, A61B 50/00

(54) **ENCLOSURE FOR A WEARABLE ACOUSTIC MONITORING DEVICE**
GEHÄUSE FÜR EINE AM KÖRPER TRAGBARE AKUSTISCHE ÜBERWACHUNGSVORRICHTUNG
ENCEINTE POUR UN DISPOSITIF DE DETECTION ACOUSTIQUE PORTABLE

(30) Priority: 24.05.2018 GB 201808523
(43) Date of publication of application: 14.04.2021
(73) Proprietor: Acurable Limited, London EC1V 9EE (GB)
(72) Inventor: SYED, Anasa Imtiaz, London EC1V 9EE (GB); RODRIGUEZ-VILLEGAS, Esther, London EC1V 9EE (GB); BOWYER, Stuart, London EC1V 9EE (GB)
(74) Representative: Mishcon de Reya LLP
(86) International application number: PCT/GB2019/051306
(87) International publication number: WO 2019/224519

(56) References cited:
- WO-A1-94/25835
- CN-U- 202 699 305
- JP-A- H04 132 786
- US-A1- 2004 215 094
- US-A1- 2013 108 507
- US-A1- 2016 100 817
- US-A1- 2016 270 685

## Description

### FIELD

The present invention relates to an enclosure for a wearable acoustic monitoring device.

### BACKGROUND

Wearable electronic devices, intended to be used for extended physiological signal monitoring, are typically heavily constrained in terms of size and functionality. A wearable device has to be small and light so that wearing it does not feel like a nuisance for the user. But making the device small, whilst requiring long term operation imposes challenging constraints in terms of the choice and distribution of electronic components. Among other factors, size, battery lifetime, electronic bandwidth and noise are conflicting requirements, and all of them need to be considered together to optimise design trade-offs. Ultimately, the overall volume of the device is going to be dominated by the power source, and this is in turn going to condition the device's shape and volume. But in addition to this, wearable devices used to acquire signals that could be used for medical diagnosis are further constrained by safety and regulatory requirements. These requirements also condition how the system is designed, the choice and combination of electronic components as well as how the wearable device is assembled.

The specific type of wearable device this invention relates to is a device intended to monitor body sounds. Body sounds can be used among others to help to diagnose a large number of diseases. Examples, include but are not limited to chronic respiratory diseases, such as Chronic Obstructive Pulmonary Disease (COPD) and asthma.

Acquiring body sounds effectively, however, is very challenging from an engineering point of view, and it becomes even more challenging when trying to do so with a very small device which should operate continuously for a long period of time. The acoustic signals generated by the different body organs are highly attenuated by the point they reach the body surface. In addition to that, when trying to capture the signals of interest, they cannot be easily isolated from other acoustic sources, resulting in interfering signals that are, in most cases, significantly stronger than the signal of interest. Furthermore, after transduction, the signals can be corrupted by other electrical signals (electronic noise as well as interference) and the local body region where the device is going to be placed will be very different from person to person. Hence, for a wearable acoustic monitor to deliver the best performance, it is of paramount importance to optimise the acoustic transmission of the signal of interest, as well as to try to minimise the potential transmission of acoustic and electrical interfering signals/noise. But all of this needs to be done taking into account the constraints on design size, shape, duration of monitoring and usability in general. Hence, a spatial distribution of components has to be chosen that takes all of this into account. The enclosure plays a crucial role in optimising these trade-offs.

Manufacturers recommend that to optimise acoustic transmission, an "acoustic chamber" is formed that only slightly exceeds the dimensions of the microphone acoustic port, and to design the surroundings in an acoustically sealed manner to avoid acoustic leakage. A totally flat enclosure with a hole at the location of the microphone, of similar dimensions to the latter would seem like the obvious way to design it, if MEMS microphones manufacturers' recommendations were followed, and would also be beneficial in terms of size and usability. However, because of the variety of characteristics of body tissues and local shapes, having a flat enclosure significantly attenuates the transmission of the acoustic signals in some subjects. A non-flat area can also be a problem because it may diminish the effectiveness of the means of attachment to the body.

The present invention seeks to address the aforementioned problems.

Prior art document US 2016/100817 discloses an enclosure for a wearable acoustic monitoring device, the enclosure comprising: a hollow body portion defined by a sidewall and top wall; and a substantially planar base portion configured to interface with the hollow body portion thus forming a cavity therebetween, the base portion comprising a flat surface and an acoustic port through the flat surface, for acoustic communication from the body surface to an acoustic sensor housed within the cavity.

### SUMMARY

The present invention relates to an enclosure for a wearable acoustic monitoring device, the enclosure comprising: a hollow body portion defined by a sidewall and top wall; and a substantially planar base portion configured to interface with the hollow body portion thus forming a cavity therebetween, wherein, the base portion comprises a flat engagement surface, for engagement with a body surface, and an acoustic port through the flat engagement surface, for acoustic communication from the body surface to an acoustic sensor housed within the cavity, and, wherein, the acoustic port is defined by a depression in the flat engagement surface of the base portion such that the acoustic sensor is spaced apart from the body surface, in use.

A protective enclosure, which simultaneously optimises the acoustic, electrical and usability characteristics, for an acoustic monitor is provided. The body portion of the enclosure prevents direct contact between the user and the main bulk of the electronics. An acoustic monitor will have one or several acoustic transducers to sense audio signals, such as MEMS microphones. The enclosure has to be created in such a way that it optimises the acoustic signal transmission from the body to at least one of such microphones, whilst minimising the very short range acoustic noises, and still providing the safety protection characteristics, particularly if the monitor is intended to be used as a medical device. Hence, it is important that air leakage gaps are avoided, not just in the space between the body and the enclosure, but also internal air leakages. It is also important to have a sufficient air gap between the body and the sensor, taking into account different skin and body shapes characteristics.

The depression in the flat engagement surface of the base portion may comprise a recess having a perimeter or circumference larger than the acoustic port. Alternatively, the depression in the flat engagement surface of the base portion may comprise an internally facing projection having a channel therethrough. Alternatively, the depression in the flat engagement surface of the base portion may comprise inwardly projecting sidewalls configured to engage with a part of the body portion. Each of the above configurations provides a good acoustic connection between a user's body and the acoustic monitoring circuitry located within the housing.

The body portion may comprise one or more internal support members projecting radially from the top of the body portion and/or one or more internal support members projecting vertically from the sidewall of the body portion. The radial internal support members and vertical internal support members increase the strength and rigidity of the body portion of the enclosure and also provide mechanical constraints for one or more internal electronic components.

The base portion may comprise a plurality of internal support members projecting radially from a central hub. The radial support members provide increase resistance to compressive forces applied axially to the enclosure and thus aid in protecting the electronic circuitry housed within the enclosure.

The body portion may further comprise an internal key projecting vertically from the sidewall thereof and the base portion may further comprise a corresponding location socket cooperable with the key of the body portion. This configuration allows for ease of assembly of the enclosure by enabling a manufacturing operative to easily align the body portion of the enclosure with the base portion. The key and socket also provide increased resistance to torsional force applied to the enclosure.

The body portion and the base portion may interface by way of a one-way snap fit interface that allows assembly only but which requires destruction of, or damage to, the enclosure to separate the body portion and the base portion once the enclosure is assembled.

Another aspect of the present disclosure, but which does not fall within the scope of the claims, provides an adhesive article comprising a pad having a first side coated with an adhesive and a second side coated with an adhesive, a first removable liner covering the first side and a second removable liner covering the second side, wherein each of the first and second removable liners comprise respective tabs that extend beyond the pad, and wherein the tabs are orientated such that they do not substantially overlap.

The orientation of the tabs enables a user to easily self apply the adhesive article to the enclosure and then the enclosure to the user. By avoiding overlap of the tabs it is easy for the user to grasp the appropriate tab to remove the first removable liner or second removable liner without also grasping the other removable liner.

Another aspect of the invention provides transportation apparatus for a wearable acoustic monitoring device comprising a primary enclosure comprising: a hollow body portion defined by a sidewall and top wall; and a substantially planar base portion configured to interface with the hollow body portion thus forming a cavity therebetween, wherein, the base portion comprises a flat engagement surface, for engagement with a body surface, and an acoustic port through the flat engagement surface, for acoustic communication from the body surface to an acoustic sensor housed within the cavity, and, wherein, the acoustic port is defined by a depression in the flat engagement surface of the base portion such that the acoustic sensor is spaced apart from the body surface, in use; and a secondary enclosure comprising a top part and a bottom part hingedly connected, an insulating layer provided in each of the top and bottom parts of the secondary enclosure and recessed to receive a portion of the primary enclosure.

Another aspect of the invention provides a wearable acoustic monitoring device comprising an enclosure comprising: hollow body portion defined by a sidewall and top wall; and a substantially planar base portion configured to interface with the hollow body portion thus forming a cavity therebetween, wherein, the base portion comprises a flat engagement surface, for engagement with a body surface, and an acoustic port through the flat engagement surface, for acoustic communication from the body surface to an acoustic sensor housed within the cavity, and, wherein, the acoustic port is defined by a depression in the flat engagement surface of the base portion such that the acoustic sensor is spaced apart from the body surface, in use; one or more acoustic transducers within the enclosure and acoustically coupled to the communication means; and a means of fastening the device to a user.

### FIGURES

In the figures:
FIG. 1 is a perspective view of an embodiment of the invention shown from a side.
FIG. 2 is a perspective view of an embodiment of the invention shown from the bottom.
FIG. 3 is a section view through the centre of an embodiment of the invention.
FIG. 4 is a perspective view of a body enclosure of an embodiment of the invention shown from the bottom.
FIG. 5 is a perspective view of a base enclosure of an embodiment of the invention shown from the top.
FIG. 6 is a perspective view of a base enclosure of an embodiment of the invention shown from the bottom.
FIG. 7 is an exploded view of an embodiment of the invention.
FIG. 8 is a section view through the centre of an enclosure from an embodiment of the invention with a modified assembly method.
FIG. 9 is a section view through the centre of an enclosure from an embodiment of the invention with a modified well in the bottom.
FIG. 10 is a section view through the centre of an enclosure from an embodiment of the invention with a further modified well in the bottom.
FIG. 11 is a perspective view of a body enclosure of an embodiment of the invention with increased strength and rigidity, shown from the bottom.
FIG. 12 is a perspective view of a base enclosure of an embodiment of the invention with increased strength and rigidity, shown from the top.
FIG. 13 is a section view of the body enclosure, base enclosure and internal electronics of an embodiment of the invention with increased strength and rigidity.
FIG. 14 is a section view through the centre of a base enclosure of an embodiment of the invention with an optical port.
FIG. 15 is a section view through the centre of a base enclosure of an embodiment of the invention with an integrated switch mechanism.
FIG. 16 is a perspective view of a body enclosure of an embodiment of the invention with rotation fixing mechanism shown from the bottom.
FIG. 17 is a perspective view of a base enclosure of an embodiment of the invention with rotation fixing mechanism shown from the top.
FIG. 18 is a section view through the centre of the body enclosure, base enclosure and internal electronics of an embodiment of the invention with a sealing gasket between the well and electronic components.
FIG. 19 is a perspective view of an embodiment of the invention with secondary mechanical insulation.
FIG. 20 is an exploded view of an adhesive pad according to the present disclosure.
FIG. 21 is an exploded view of an adhesive pad with internal hold according to the present disclosure.
FIG. 22 is a perspective view of an embodiment of the invention with additional fixing point and a strap.

### DESCRIPTION

Embodiments of enclosures according to the present invention are illustrated in the figures.

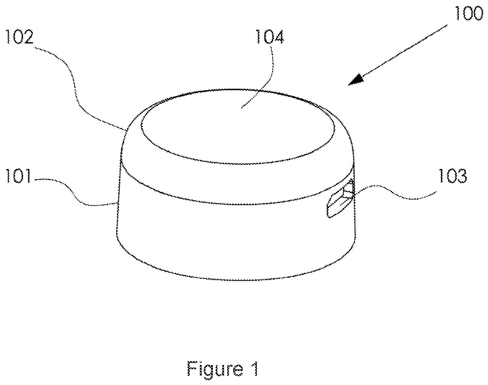

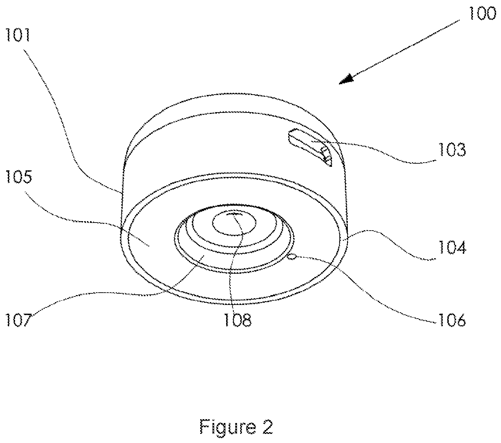

A first embodiment of the invention is shown in general terms in Fig. 1. An enclosure 100 comprises a body portion 104 and a base portion (not shown in Fig. 1). The body portion is defined by a non-vertical annular side wall 101 joined to a planar top surface by a curved top edge 102. A charging/data transfer interface 103 is provided through the annular side wall 101. As shown in Fig. 2, the base portion 105 fits within the body portion 104 to provide a planar bottom surface. A recess or depression 107 is provided within the base portion to enable an acoustic port 108 to be spaced apart from a surface to be contacted. The base of the enclosure also has one or more entry ports 106 for access to internal components (e.g. reset switches) without having to disassemble the enclosure 100.

The body portion 104 and base portion 105 are attached together with an annular snap-fit mechanism 114/117 that allows for easy assembly during manufacture but limits accessibility to the internal components when in use or when dropped or struck. The annular snap-fit geometry 114/117 and lack of graspable features on the base portion 105 are created in such a way that engagement is possible with a limited amount of force, however, disengagement requires the destruction of all or part of the enclosure 100.

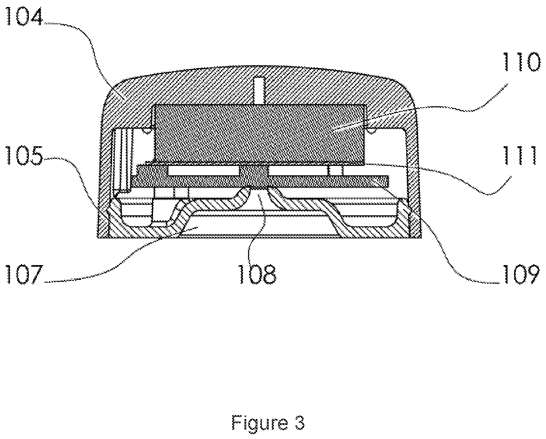

The enclosure 100 is designed to house the electronic components 109 of a very small acoustic monitoring device. A typical device, as shown in cross section in Fig.3, will be formed of one or more acoustic transducers, such as MEMS microphones, associated electronic circuitry 109 and a power source 110. In embodiments provided with multiple acoustic transducers, some of the acoustic transducers will aim to "capture" the acoustic signal of interest, whereas others will aim to "capture" acoustic interference. The efficiency of these processes will be affected by the specific location of each transducer (this will determine the relative position to the acoustic source of interest): the relative position of each acoustic transducer with respect to one another (for example the closer they are together the more likely the signal of interest for that transducer is to be corrupted by signals from other transducers), and the internal acoustic surroundings (different air gaps are going to lead to differences in transmission of acoustic signals). In embodiments of the invention, a hole facing the transducer sensing port (although several holes could be applied to a plurality of them) is present at the bottom of the base portion 105 of the enclosure. The hole will be of such dimension, that any internal active track that could violate a safety constraint as per 60601-1 would not be an accessible part. In addition to this, the attachment area is distributed between an all-around attachment area, and a hollow well, or recess, with typical height could be 2.5 mm. Note that the shape of this hollow region does not need to be circular. Surrounding the recess is a flat area configured to enable optimum attachment to a user's body

The different acoustic transducers will be followed by conditioning circuitry 109 (mostly amplifiers and filters, although there could also be algorithms implemented in analog processing the signal), prior to analog-to-digital conversion, to prepare the signal (raw or processed) for wireless transmission. A microcontroller (or equivalent chip) will provide the control signals for different circuit blocks as well as the transmitter chip. In addition to all of this, some peripheral circuitry might be required, such as voltage regulators to provide biasing signals, LEDs to provide battery status, and charging and protection circuitry, passive components for noise and interference reduction as well as to optimise transmission, an antenna, and a power source 110 (such as a rechargeable battery).

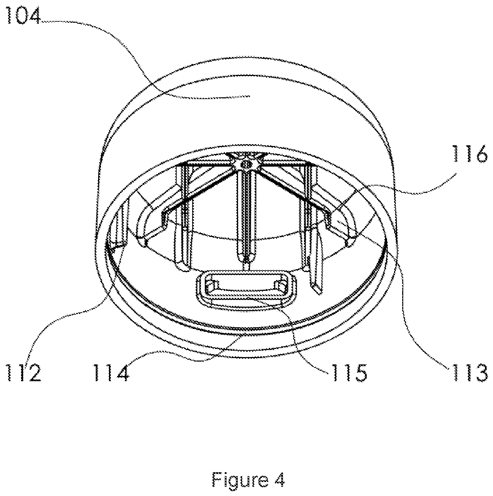

The body portion 104, as shown in Fig. 4, has multiple ribs 113 extending horizontally across the inside thereof. These ribs increase the strength and rigidity of the enclosure and provide mechanical constraints for one or more of the internal components. During assembly, electronic components such as batteries 110 are constrained within the body enclosure with profiled sections 116 included in the internal ribs. The body enclosure also includes several vertical struts 112 attached to the inside walls which serve multiple purposes. Firstly, these struts 112 are to support and constrain the internal circuitry 109 (printed circuit boards). Some of the struts 112 constrain the circuitry 109 to correct horizontal plane within the enclosure 100 and some of the struts 112 constrain the rotation of the circuitry 109 within the enclosure 100. This constraining of the circuitry 109 ensures that all components are aligned correctly to perform their respective function, for example the alignment of the programming or charging ports with the opening 103 in the enclosure 100. Secondly, these struts 112 are to strengthen and stiffen the enclosure 100. Importantly, the vertical geometry of these struts 112 minimises their mass and volume so that the overall mass of the enclosure 100 is reduced while also avoiding obstruction of the wireless communication any more than is necessary.

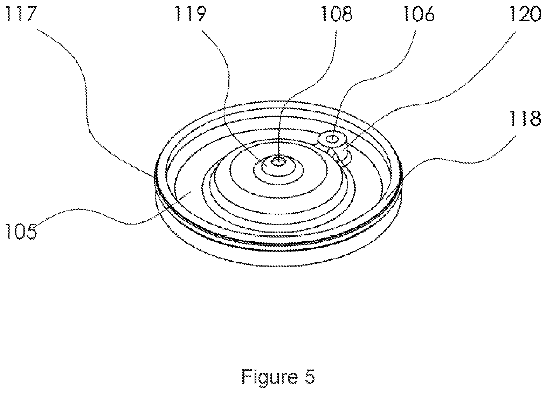

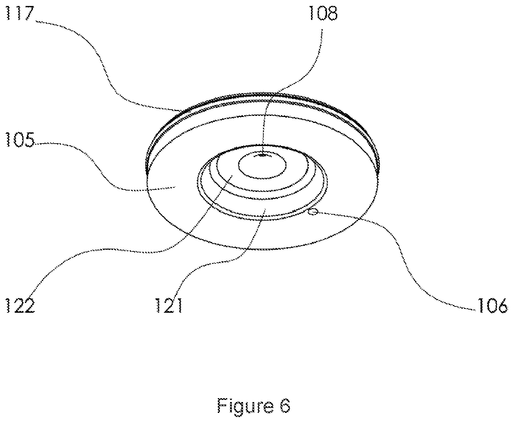

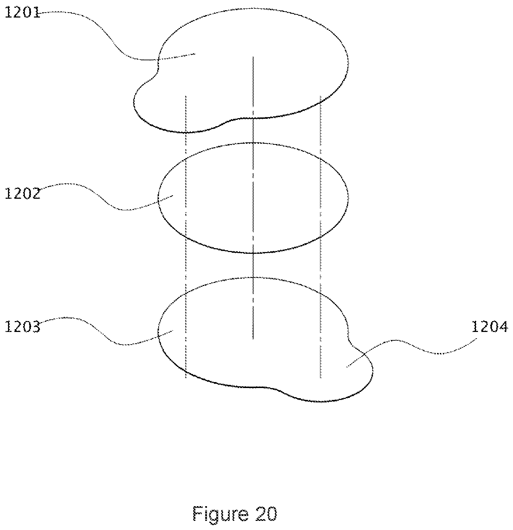

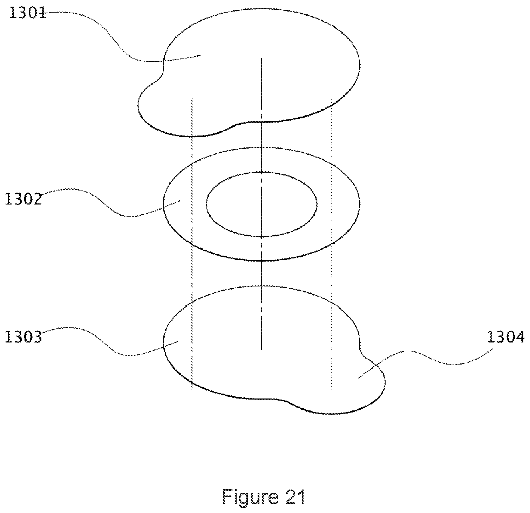

The base portion, as shown in Figs. 5 and 6, includes a wide flat interface area for the attachment of adhesive pads (as shown in figures 20 and 21. The recess in the base portion, can have angled or vertical side walls 121 and a flat or curved top surface 122 that leads into a central hole 108 that interfaces with the electronic componentry 109. The recess is part of the chamber, (when in contact with a surface) and its dimensions are much larger than the dimensions of the acoustic port of the acoustic transducer.

The interface between the recess and the internal electronic circuitry 109 may be formed with a lip 119 that presses against the electronic circuitry, or other internal components. The geometry and location of this lip 119 is designed in such a way that it ensures a good acoustic transmission from the base portion to the sensing circuitry 109. The geometry and location of this lip 119 also function as preload/elastic clamping mechanism to constrain the position and orientations of the internal components. An elastic or compressible sheet material 111 can be included between different components of the electronic assembly to assist in clamping the internal components and accommodate for any small manufacturing variations in the component's geometries. This material can also function as a mechanical, electrical or thermal insulator.

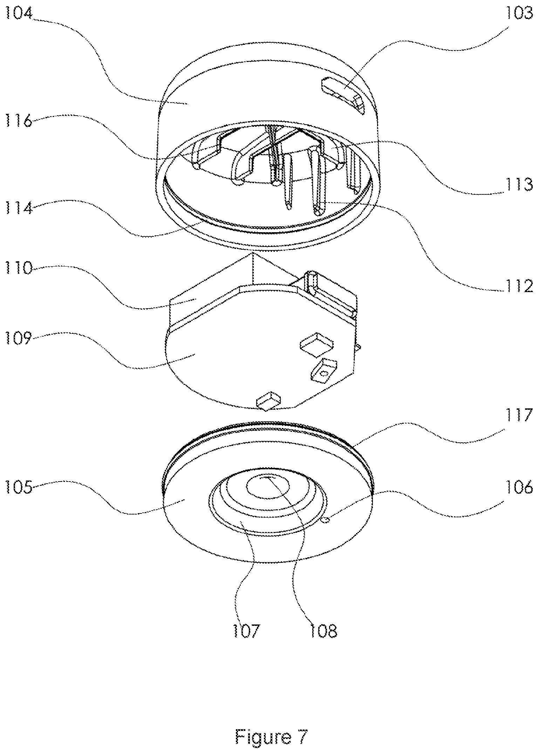

As shown in Fig. 7, the internal components including the internal circuitry 109 and power source 110, are pre-configured into a single unit for ease of insertion into the body portion 104 of the enclosure. Once the internal components are constrained in the correct location by the vertical struts 112 and horizontal ribs 113, the base portion 105 is snap fitted to the body portion 104.

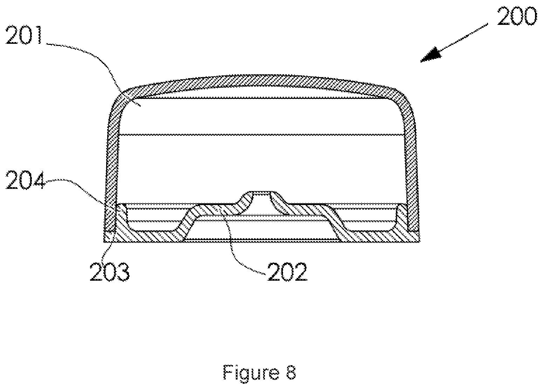

Another embodiment of the invention is shown in cross-section in Fig. 8. An enclosure 200 comprises a body portion 201 and a base portion 202 secured together through surface bonding techniques such as ultrasonic welding or adhesive. The base portion 202 includes an annular projection 204 extending perpendicularly from the contact surface of the base portion 202. The annular projection 204 is configured to either sit inside the body portion 201 or around outside of the body portion 201 (Fig. 8 shows the annular projection 204 sitting inside the body portion 201). This configuration allows for the accurate alignment of the body and base portions of the enclosure to ensure correct signal transmission and functionality, and provides an increased surface area 203 for the surface bonding method to engage with, thus providing strength and resisting separation of the body portion 201 and base portion 202.

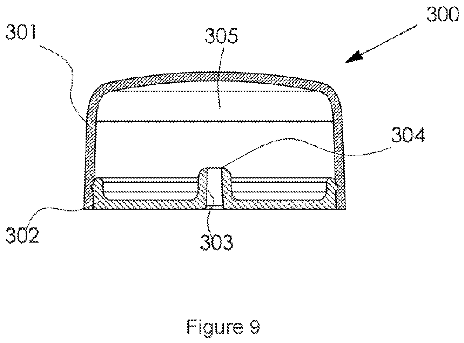

Another embodiment of the invention is shown in cross-section in Fig. 9. An enclosure 300 comprises a body portion 301 and a base portion 302 secured together either through a snap-fit interface or surface bonding techniques. The base portion 302 includes a substantially planar surface and an elongate inward depression having a channel 303 therethrough. The channel 303 has either substantially straight or angled sidewalls configured to direct air pressure from an acoustic event directly into the acoustic sensing circuitry 304. This embodiment maximises the acoustic transmission efficiency from the user to the internal sensing electronics 304.

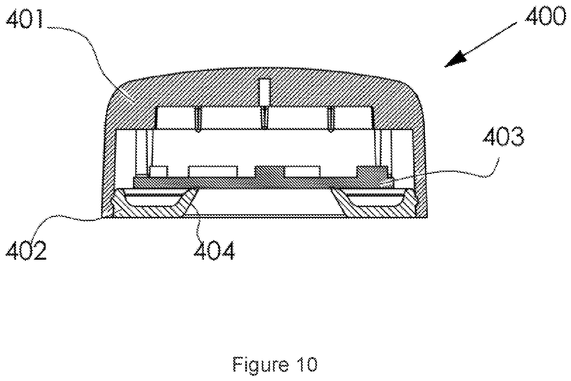

Another embodiment of the invention is shown in cross-section in Fig. 10. An enclosure 400 comprises a body portion 401 and a base portion 402 secured together either through a snap-fit interface or surface bonding techniques. The base portion 402 includes a recess with angled sidewalls 404 that defines a large opening configured to interface directly with the acoustic sensing circuitry 403. This embodiment provides a space efficient stepped profile for the depression by combining with the bottom surface of the internal electronic circuit board 403. The side walls 404 of the depression are part of the base portion 402, and press against the electronic circuit board 403 at a larger diameter than in embodiment 100. The depression formed by the combination of the base portion 402 and the internal electronic circuit board 403 can have the same air volume characteristics as in embodiment 100, with a reduced vertical dimension, i.e less than 2.5mm.

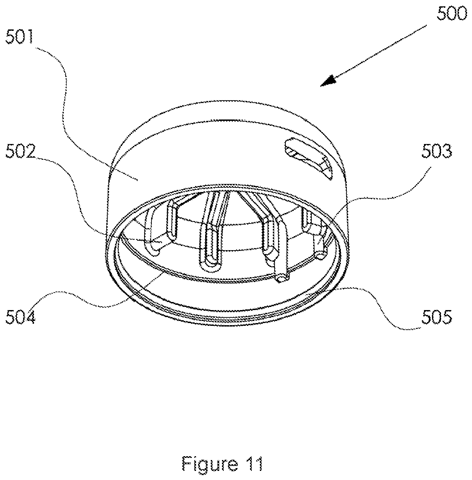

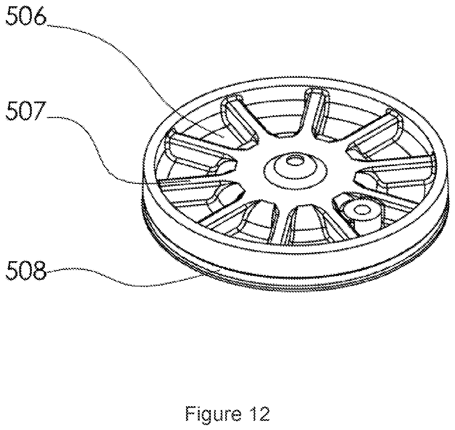

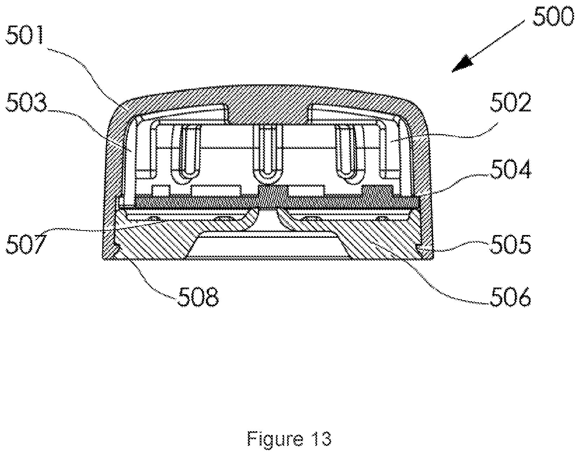

Another embodiment of the invention is shown in Figs. 11, 12 and 13. An enclosure 500 has several features to enhance strength and allow the enclosure 500 to withstand greater impact and crushing. This embodiment has an increased number and depth of ribs 502 inside the body portion 501, compared to embodiment 100, and has the addition of radial ribs 507 in the base portion 506. Additionally, this embodiment has a stepped lip 504 on the inside of the body portion 501 to constrain the internal electronic circuitry. This stepped lip 504 provides for a significantly increased contact area with the circuitry and therefore greater constraining. The base portion 506 in this embodiment has an increased sized outer insert that presses against the base of the internal electronic circuitry. Combining the increased sized outer insert of the base portion 506 with the stepped lip 504 on the inside of the body portion 501 creates a mechanical reinforcement that distributes impact and crush loads all the way through the enclosure 500. This embodiment also includes increased sized support struts 502, as compared to embodiment 100, in the body portion 501 to provide a greater resistance to crushing loads. Embodiment 500 includes a greater strength annular snap-fit design to ensure that the components do not separate during loading. This snap-fit is provided by an annular groove 508 in the base portion and an annular protrusion 505 in sidewall of the body portion 501 to avoid any thin wall sections in the externally exposed body portion 501. Additionally, the snap-fit protrusion 505 has an asymmetric hooked profile that requires significantly more force to disassemble than assemble.

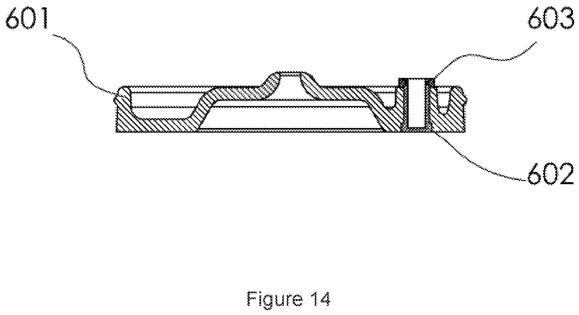

Another embodiment of the invention is shown in Fig. 14. An enclosure 600 includes a base portion 601 that has a covered port 602 to allow light transmission from internal components to the bottom surface of the base portion 601 while maintaining a level of ingress protection. The covered port 602 is created by the combination of a transmissive optical device (e.g. lens) 603, a corresponding hole through the enclosure base and a method of retaining the optical device within the enclosure 600. The method of retaining the optical device could include adhesive, retaining clips, retaining tabs, and a snap-fit mechanism or by alignment of the optical device with other internal components such that it is constrained within the enclosure. This design can be configured so that the bottom surface of the transmissive optic 603 is flush with the bottom surface of the enclosure base to ensure correct adhesion and minimise any corruption to certain optical signals. The acoustic monitor may have indicator lights that indicate various items, such as power, communication or other status items. The enclosure 600 might be designed with a material and thickness that without modification allows for the light to pass through, or the material might be thinned out in regions in which there is no violation of insulator strength for safety reasons, to facilitate the light transmission from the source, or a different translucent/transparent material might be used, as a separate part that could be assembled during fabrication. This "light optimisation area" (optical window) will generally be located either on the bottom area, or on the sides of the enclosure. Their specific location will depend on the existing internal air gaps, providing a light path from the light source to the enclosure 600, as well as on the distance from different current tracks to the specific location in the enclosure 600.

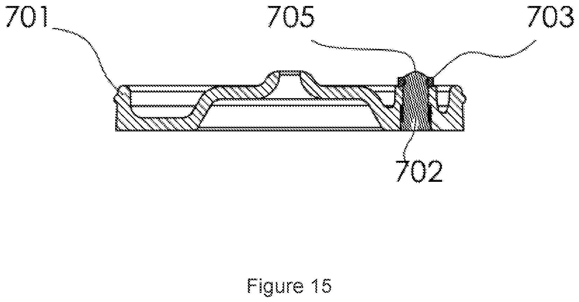

Another embodiment of the invention is shown in Fig. 15. An enclosure 700 includes a base portion 701 that has an incorporated button or switch 702 for user interfacing with internal electronic componentry while maintaining a level of ingress protection. The button or switch 702 is located into a hole in the base portion 701. The button 702 can be retained with a clip or similar mechanism 703 and can be fitted with a spring, other tensioning mechanism or sealing mechanism around its diameter. The button body is free to move vertically with a prescribed range and can be sprung loaded to return to the bottom position. The interfacing point 705 can be shaped in any required way so that, when a user pushes the body upwards, it will interface with and engage the internal electronic circuitry, as required. This action could also be formed with a contactless switch by fitting inductive or similar components to the internal surface of the base portion 701.

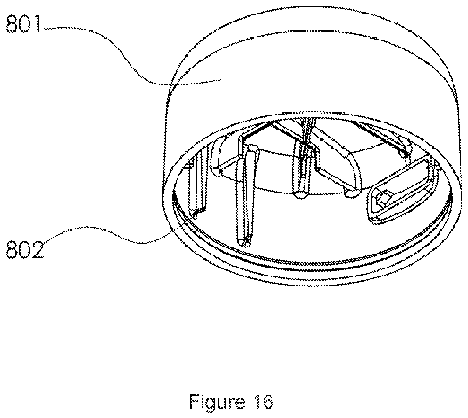

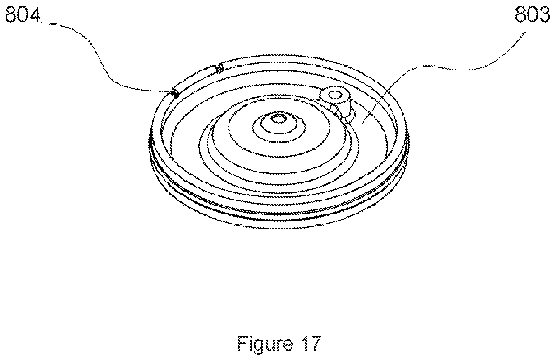

Another embodiment of the invention is shown in Figs. 16 and 17. An enclosure 800 comprises a keying mechanism that aids in assembly and manufacture, and ensures that all ports on the enclosure base are accurately aligned with respective receptacles, components or sensors in the internal electronic system, whilst also allowing for enclosure mass production by moulding and maintaining water ingress protection. The keying mechanism of the body portion 801 is created with one or more extended struts 802 that run from the top of the body portion 801 to a small distance from the bottom of the body portion 801. The base portion 803 has a corresponding number of U-shaped cut-outs 804 on the top of it, at the edge and at a precisely defined angle. During assembly, the U-shaped cut-outs 804 on the base portion 803 engage with the extended struts 802 on the body portion 801, constraining the rotation of the base portion 803 relative to the body portion 801, thus aligning all ports as required.

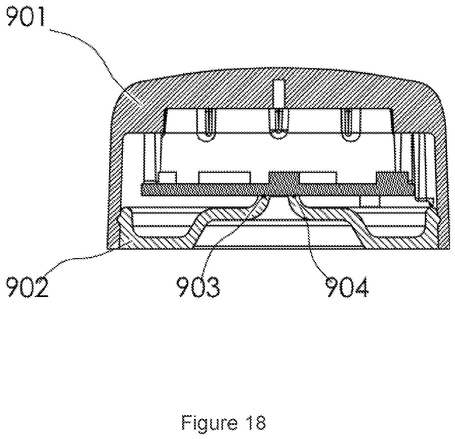

Another embodiment of the invention is shown in Fig. 18. An enclosure 900 includes a body portion 901 and a base portion 902. The base portion 902 incorporates a recess in a raised lip 903 that interfaces with the electronic circuit board to allow the inclusion, constraining and effective operation of a sealing gasket or O-ring 904 between them. This arrangement minimises the possibility of pressure or air loss from the internal volume of the enclosure, thus maximising the transmission of acoustic signals from the user to the electronic sensing components.

Distributing all these components spatially for optimum performance is not a trivial task because of the complex set of different electrical, physical, usability and physiological trade-offs that have to be taken simultaneously into account. For example, the type of application for which a monitor of this kind would be most beneficial would be one requiring long term continuous monitoring. The uninterrupted duration of monitoring is however limited because of the duration of the power source. But, if choosing a battery, the duration of the power source depends both of the chemistry of the battery, the nature of the cell (primary or secondary), and its volume. The nature of the cell will have important usability implications, since secondary cells have less capacity (energy per unit of volume) but they, however, allow for the system to be recharged. The volume will affect the size of the system, as well as its weight. The result of all of this is that the battery will be the dominating component in the volume of the system. However, in most case scenarios, the shape of the batteries is fixed (customisation might be possible but this results on a significant manufacturing cost), and hence this is going to severely limit the spatial distribution of other components. But there are components that also have their own spatial requirements. The antenna is an example of those. Depending on the antenna choice a trade-off has to be found taking into account the surface area occupied by it, the gain, and the space around it that needs to be left component-free. But in addition to that, because of the size of the system, the distribution of components is always going to have an effect in the transmission that needs to be accounted for. In addition to all of this, the positioning of the transducers is going to heavily influence their effective signal to noise ratio (i.e. this would be understood as the ratio between the larger signal they can detect and the noise floor, considering that this noise floor would also account in certain instances for interference introduced by other acoustic signals). The design of the enclosure plays a very important role on getting these trade-offs right. The enclosure can significantly affect the transmission of the different acoustic signals (both, body signals of interest as well as artefact that need to be sensed so that they can be later eliminated); can "fix" the spatial location of certain components (such as batteries) in an optimum way without having to rely on special internal connectors which would compromise other component's spatial distribution; can eliminate the need of certain battery/communications or other indicators; can facilitate resetting (or changing the status) of the system; can eliminate the need of certain means of user protection which would impact on some important system trade-offs: can protect the system; can facilitate safe battery charging; and can eliminate the need of cumbersome means of user attachment.

Each part of the enclosure may be fabricated from medical grade acrylonitrile butadiene styrene (ABS) with a thickness of 1 mm. But those skilled in the art may use other types of materials without deviating from the present invention. A polished finished for the enclosure would be desirable, both for aesthetic as well as performance factor. A rugged surface finish will generate stronger acoustic artefacts, leading to more signal corruption. However, a polished finish also leads to higher production costs.

Although, with appropriate means of body attachment the acoustic transmission of body sounds will be optimised, out of the body sounds can also be picked up by the transducer. Because of this, an array (one or several) microphone might be arranged in the printed circuit board to sense those environmental noises, in order to facilitate processing and elimination. The sensing port of those transducers will generally be facing the other side of the PCB, so that they will not pick up body signals, whilst picking up noise. The enclosure might be designed to guarantee an air gap between those ports and the surface, to facilitate signal transmission.

The enclosure may have internal reinforcements in order to minimise the probability of breaking into more than one piece in the situation of a strong impact, which would allow access to electrical parts compromising safety. This internal reinforcement can simultaneously be used to guide the assembly and fix some components position to minimise hazards cause by vibrations, whilst also guaranteeing the air gaps mentioned above. An example of such is shown in embodiment 500.

The enclosure may be custom coloured and could include any number or combination of logos, labels or graphical designs. The logos, labels or designs could be included into the enclosure by the adhesion or attachment of any other material, such as paint or vinyl, or by the contouring of the enclosure's surface itself, such as embossing or engraving.

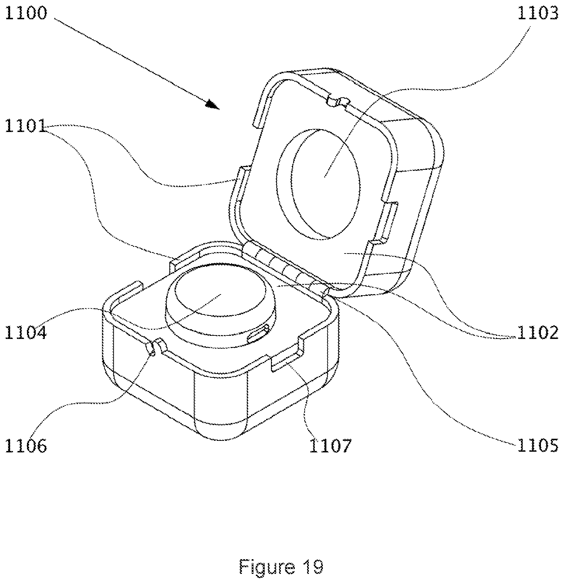

Another embodiment of the invention is shown in Fig. 19. A secondary enclosure 1100 enables an enclosure as described in relation to Figs. 1 to 18 (referred to in this paragraph as a primary enclosure) to be stored and transported in such a way that it has a significantly increased resistance to mechanical loading, impact and vibration. The secondary enclosure is formed from an impact resistant outer shell 1101, manufactured from a material such as ABS, and a mechanical insulating layer 1102, manufactured from a material such as low-density foam. A recess 1103 is included in both halves of the mechanical insulating layer to allow the primary enclosure 1104 to sit securely. The outer shell can be formed from a hinged design 1105 with a securing mechanism 1106 to keep the two parts of the secondary enclosure together and secure the primary enclosure in place. Additionally, secondary holes 1107 can be placed in the side of the secondary enclosure to allow the application of cables or other connectors to the ports on the primary enclosure. This could be used for, for example, charging device batteries while the primary enclosure is inside the protective secondary enclosure.

The enclosure must also have adequate characteristics so that it can be properly attached to a person's body without modifying the acoustic characteristics, and minimising the risk of de-attachment. Attachment can be achieved by attaching an adhesive tape to the bottom of the base portion of the enclosure (when the liner is removed, the adhesive part can be put in contact with a person's body). By doing it this way, the adhesive can serve a multiple role: keeping the enclosure in place and waterproofing/ dust-proofing the microphone hole. In order to allow multiple uses (and users) the adhesive part of the enclosure must be exchangeable. There are a number of ways of achieving this, but one that is found to be very effective is to have a double taped adhesive with two tabs, as shown in Fig. 20. The absence of tabs makes it very hard for users to centre the enclosure, due to the very small size of it, and remove the liners. However, this is an important requirement, due to the very small contact area left as a consequence of the well created in the centre of the enclosure, to avoid the attenuation of the signal caused mostly by loose skin or tissue in people with larger necks.

The user would change the adhesive by using a two-sided adhesive pad with liners designed for usability of attachment to the enclosure and attachment to the user. Embodiment 1200 of the adhesive and liners is shown in Fig. 20. The liners 1201/1203 are designed with easy manipulation tabs 1204 that protrude out past the adhesive tape 1202 that are placed at an angle to each other (e.g. 90 degrees) and each are made from materials that have a different release strength to the other to allow for control over which liner will be removed first, so that clearer instructions can be given to the user. The angle of the tabs on the liner is important for ensuring easy peeling by the user. Once the first liner has been removed, the device can be placed accurately on top of the adhesive tape. After this, the user can remove the second liner by pulling on the remaining tab and attaching the enclosure to themselves. After use, the adhesive tape can be either slightly larger in size than the enclosure or have an extra tab to allow easy manipulation of the adhesive tape by the user and removal from the enclosure.

Another embodiment of the adhesive 1300 as shown in Fig. 21 has a central hole 1305 on the adhesive tape to improve the acoustic transmission into the hollow well in the base enclosure.

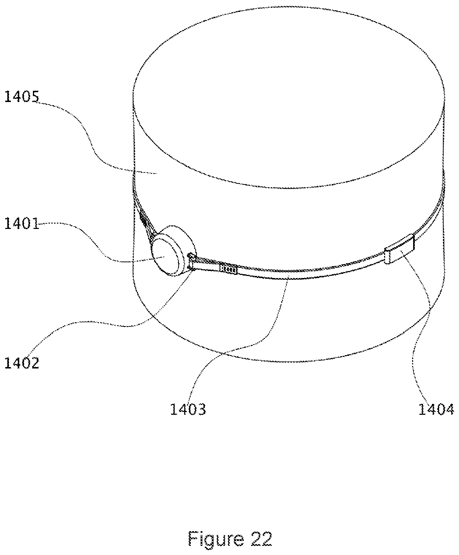

Because this is intended for very small monitoring systems, in the specific case of paediatric application, the system itself can constitute a suffocation hazard if attached only with the adhesive/surface glue or alternative method, in which a child could take it off and put it in their mouth. In this case, a strapping mechanism, as shown in Fig. 22, may be added to the enclosure reduce the hazard. The enclosure 1400 has additional mounting points 1402 on either side of the body portion 1401 for the permanent attachment of a restraining strap 1403. This strap allows for additional security of the attachment of the enclosure to the body of a user and minimises the risk of a child removing the enclosure and inserting the enclosure into its mouth. The strap can be made of any material that will comfortably enclose the body of the user 1405 and can be attached with the use of a clip, hook-and-loop fasteners or any other form of strap fastener 1404.

## Claims

1. An enclosure for a wearable acoustic monitoring device, the enclosure comprising:
a hollow body portion defined by a sidewall and top wall; and
a substantially planar base portion configured to interface with the hollow body portion thus forming a cavity therebetween,
wherein, the base portion comprises a flat engagement surface, for engagement with a body surface, and an acoustic port through the flat engagement surface, for acoustic communication from the body surface to an acoustic sensor housed within the cavity, and,
wherein, the acoustic port is located within a depression in, or elongate channel through, the flat engagement surface of the base portion such that the acoustic sensor is spaced apart from the body surface, in use.

2. The enclosure according to claim 1, wherein the depression in the flat engagement surface of the base portion comprises a recess having a perimeter or circumference larger than the acoustic port.

3. The enclosure according to claim 1, wherein the channel through the base portion comprises an internally facing projection having a channel therethrough.

4. The enclosure according to claim 1, wherein the depression in the flat engagement surface of the base portion comprises inwardly projecting sidewalls configured to engage with a part of the body portion.

5. The enclosure according to any of claims 1 to 4, wherein the base portion comprises a mounting projection extending therefrom and the body portion comprises an internal mounting groove co-operable with the mounting projection.

6. The enclosure according to any preceding claim, wherein the body portion comprises one or more internal support members projecting radially from the top of the body portion and/or one or more internal support members projecting vertically from the sidewall of the body portion.

7. The enclosure according to any preceding claim, wherein the base portion comprises a plurality of internal support members projecting radially from a central hub.

8. The enclosure according to any preceding claim, wherein the body portion further comprises an internal key projecting vertically from the sidewall thereof and the base portion further comprises a location socket cooperable with the key of the body portion.

9. The enclosure according to any preceding claim, wherein the body portion and the base portion interface by way of a one-way snap fit interface that allows assembly only but which requires destruction of, or damage to, the enclosure to separate the body portion and the base portion once the enclosure is assembled.

10. A kit comprising a transportation apparatus and a wearable acoustic monitoring device comprising the enclosure according to claim 1, wherein the transportation apparatus is configured for storing and transporting the wearable acoustic monitoring device, the transportation apparatus comnrisins a secondary enclosure comprising a top part and a bottom part hingedly connected, an insulating layer provided in each of the top and bottom parts of the secondary enclosure and recessed to receive a portion of the primary enclosure.

11. A wearable acoustic monitoring device comprising:
an enclosure according to claim 1;
one or more acoustic transducers within the enclosure and acoustically coupled to the acoustic port; and
a means of fastening the device to a user.

12. The wearable acoustic monitoring device according to claim 11, wherein the means of fastening the device to a user comprises an adhesive article comprising a planar pad having a first side coated with an adhesive and a second side coated with an adhesive, a first removable liner covering the first side and a second removable liner covering the second side, wherein each of the first and second removable liners comprise a tab that extends beyond the pad, and wherein the tabs are orientated such that they do not substantially overlap.

13. The wearable acoustic monitoring device according to any one of claims 11 or 12, comprising at least two acoustic transducers housed within an enclosure, wherein one acoustic transducer is configured to identify and record a target acoustic signal and one acoustic transducer is configured to identify and record background acoustic signals.

14. The wearable acoustic monitoring device according to claim 13 further comprising a processor for removing background acoustic signals and isolating the target acoustic signal.

## Patentansprüche

1. Gehäuse für eine tragbare akustische Überwachungsvorrichtung, wobei das Gehäuse Folgendes umfasst:
einen Hohlkörperabschnitt, der durch eine Seitenwand und eine obere Wand definiert ist, und
einen im Wesentlichen ebenen Basisabschnitt, der konfiguriert ist, um eine Schnittstelle mit dem Hohlkörperabschnitt zu bilden und dadurch einen Hohlraum dazwischen zu formen,
wobei der Basisabschnitt eine flache Eingriffsfläche zum Eingriff mit einer Körperfläche und einen akustischen Anschluss durch die flache Eingriffsfläche zur akustischen Kommunikation von der Körperfläche zu einem akustischen Sensor, der innerhalb des Hohlraums untergebracht ist, umfasst, und
wobei sich der akustische Anschluss innerhalb einer Vertiefung in der oder eines länglichen Kanals durch die flache(n) Eingriffsfläche des Basisabschnittes befindet, sodass der akustische Sensor in Verwendung von der Körperfläche beabstandet ist.

2. Gehäuse nach Anspruch 1, wobei die Vertiefung in der flachen Eingriffsfläche des Basisabschnittes eine Aussparung mit einem Umkreis oder Umfang umfasst, der größer als der akustische Anschluss ist.

3. Gehäuse nach Anspruch 1, wobei der Kanal durch den Basisabschnitt einen nach innen gerichteten Vorsprung mit einem Kanal dort hindurch umfasst.

4. Gehäuse nach Anspruch 1, wobei die Vertiefung in der flachen Eingriffsfläche des Basisabschnittes nach innen vorstehende Seitenwände umfasst, die konfiguriert sind, um einen Teil des Körperabschnittes in Eingriff zu nehmen.

5. Gehäuse nach einem der Ansprüche 1 bis 4, wobei der Basisabschnitt einen Montagevorsprung umfasst, der sich davon erstreckt, und der Körperabschnitt eine innere Montagenut umfasst, die mit dem Montagevorsprung zusammenwirkbar ist.

6. Gehäuse nach einem vorhergehenden Anspruch, wobei der Körperabschnitt ein oder mehrere innere Stützelemente, die radial von der Oberseite des Körperabschnittes vorstehen, und/oder ein oder mehrere innere Stützelemente, die vertikal von der Seitenwand des Körperabschnittes vorstehen, umfasst.

7. Gehäuse nach einem vorhergehenden Anspruch, wobei der Basisabschnitt eine Vielzahl von inneren Stützelementen umfasst, die radial von einer zentralen Nabe vorsteht.

8. Gehäuse nach einem vorhergehenden Anspruch, wobei der Körperabschnitt ferner einen inneren Schlüssel umfasst, der vertikal von der Seitenwand davon vorsteht, und der Basisabschnitt ferner eine Positionsbuchse umfasst, die mit dem Schlüssel des Körperabschnittes zusammenwirkbar ist.

9. Gehäuse nach einem vorhergehenden Anspruch, wobei der Körperabschnitt und der Basisabschnitt über eine Einwegschnappverbindung eine Schnittstelle bilden, die nur Zusammenbau ermöglicht, aber die Zerstörung oder Beschädigung des Gehäuses erfordert, um den Körperabschnitt und den Basisabschnitt zu trennen, sobald das Gehäuse zusammengebaut ist.

10. Kit, umfassend ein Transportgerät und eine tragbare akustische Überwachungsvorrichtung, die das Gehäuse nach Anspruch 1 umfasst, wobei das Transportgerät konfiguriert ist, um die tragbare akustische Überwachungsvorrichtung aufzubewahren und zu transportieren, wobei das Transportgerät ein sekundäres Gehäuse umfasst, das einen oberen Teil und einen unteren Teil umfasst, die gelenkig verbunden sind, wobei eine Isolierschicht in jedem von dem oberen und dem unteren Teil des sekundären Gehäuses bereitgestellt und ausgespart ist, um einen Abschnitt des primären Gehäuses aufzunehmen.

11. Tragbare akustische Überwachungsvorrichtung, umfassend:
ein Gehäuse nach Anspruch 1;
einen oder mehrere akustische Wandler innerhalb des Gehäuses und akustisch an den akustischen Anschluss gekoppelt; und
ein Mittel zum Befestigen der Vorrichtung an einem Benutzer.

12. Tragbare akustische Überwachungsvorrichtung nach Anspruch 11, wobei das Mittel zum Befestigen der Vorrichtung an einem Benutzer einen Klebeartikel umfasst, der ein ebenes Polster umfasst, das eine erste Seite, die mit einem Klebstoff beschichtet ist, und eine zweite Seite, die mit einem Klebstoff beschichtet ist, aufweist, wobei ein erster entfernbarer Liner die erste Seite bedeckt und ein zweiter entfernbarer Liner die zweite Seite bedeckt, wobei jeder von dem ersten und dem zweiten entfernbaren Liner eine Lasche umfasst, die sich über das Polster hinaus erstreckt, und wobei die Laschen ausgerichtet sind, sodass sie sich im Wesentlichen nicht überlappen.

13. Tragbare akustische Überwachungsvorrichtung nach einem der Ansprüche 11 oder 12, umfassend zumindest zwei akustische Wandler, die innerhalb eines Gehäuses untergebracht sind, wobei ein akustischer Wandler konfiguriert ist, um ein akustisches Zielsignal zu identifizieren und aufzuzeichnen und ein akustischer Wandler konfiguriert ist, um akustische Hintergrundsignale zu identifizieren und aufzuzeichnen.

14. Tragbare akustische Überwachungsvorrichtung nach Anspruch 13, ferner umfassend einen Prozessor zum Entfernen von akustischen Hintergrundsignalen und Isolieren des akustischen Zielsignals.

## Revendications

1. Enceinte pour un dispositif de détection acoustique portable, l'enceinte comprenant :
une partie corps creux définie par une paroi latérale et une paroi supérieure ; et
une partie de base sensiblement plane conçue pour faire interface avec la partie corps creux formant ainsi une cavité entre elles,
ladite partie de base comprenant une surface plate de mise en prise, destinée à la mise en prise avec une surface du corps, et un orifice acoustique à travers la surface plate de mise en prise, pour une communication acoustique de la surface du corps à un capteur acoustique logé au sein de la cavité, et, ledit orifice acoustique se trouvant au sein d'une dépression dans, ou d'un canal allongé à travers, la surface plate de mise en prise de la partie de base de sorte que le capteur acoustique soit espacé de la surface du corps, lors de l'utilisation.

2. Enceinte selon la revendication 1, ladite dépression dans la surface plate de mise en prise de la partie de base comprenant un évidement comportant un périmètre ou une circonférence plus grand que l'orifice acoustique.

3. Enceinte selon la revendication 1, ledit canal à travers la partie de base comprenant une saillie orientée vers l'intérieur traversée par un canal.

4. Enceinte selon la revendication 1, ladite dépression dans la surface plate de mise en prise de la partie de base comprenant des parois latérales saillantes vers l'intérieur conçues pour se mettre en prise avec une partie de la partie corps.

5. Enceinte selon l'une quelconque des revendications 1 à 4, ladite partie de base comprenant une saillie de montage s'étendant à partir de celle-ci et ladite partie corps comprenant une rainure de montage interne pouvant coopérer avec la saillie de montage.

6. Enceinte selon l'une quelconque des revendications précédentes, ladite partie corps comprenant un ou plusieurs éléments supports internes faisant saillie radialement depuis le haut de la partie corps et/ou un ou plusieurs éléments supports internes faisant saillie verticalement depuis la paroi latérale de la partie corps.

7. Enceinte selon l'une quelconque des revendications précédentes, ladite partie de base comprenant une pluralité d'éléments supports internes faisant saillie radialement depuis un moyeu central.

8. Enceinte selon l'une quelconque des revendications précédentes, ladite partie corps comprenant en outre une clé interne faisant saillie verticalement depuis sa paroi latérale et ladite partie de base comprenant en outre une douille de positionnement pouvant coopérer avec la clé de la partie corps.

9. Enceinte selon l'une quelconque des revendications précédentes, ladite partie corps et ladite partie de base faisant interface au moyen d'une interface à encliquetage unidirectionnelle qui permet uniquement l'assemblage mais qui nécessite la destruction ou l'endommagement de l'enceinte pour séparer la partie corps et la partie de base une fois le boîtier assemblé.

10. Kit comprenant un appareil de transport et un dispositif de détection acoustique portable comprenant l'enceinte selon la revendication 1, ledit appareil de transport étant conçu pour stocker et transporter le dispositif de détection acoustique portable, ledit appareil de transport comprenant une enceinte secondaire comprenant une partie supérieure et une partie inférieure reliées de manière articulée, une couche isolante pourvue dans chacune des parties supérieure et inférieure de l'enceinte secondaire et en retrait pour recevoir une partie de l'enceinte primaire.

11. Dispositif de détection acoustique portable comprenant :
une enceinte selon la revendication 1 ;
un ou plusieurs transducteurs acoustiques au sein de l'enceinte et couplés acoustiquement à l'orifice acoustique ; et
un moyen de fixation du dispositif à un utilisateur.

12. Dispositif de détection acoustique portable selon la revendication 11, ledit moyen de fixation du dispositif à un utilisateur comprenant un article adhésif comprenant un tampon plan comportant un premier côté enduit d'un adhésif et un second côté enduit d'un adhésif, un premier revêtement amovible recouvrant le premier côté et un second revêtement amovible recouvrant le second côté, chacun des premier et second revêtements amovibles comprenant une languette qui s'étend au-delà du tampon, et lesdites languettes étant orientées de sorte qu'elles ne se chevauchent sensiblement pas.

13. Dispositif de détection acoustique portable selon l'une quelconque des revendications 11 ou 12, comprenant au moins deux transducteurs acoustiques logés au sein d'une enceinte, un transducteur acoustique étant conçu pour identifier et enregistrer un signal acoustique cible et un transducteur acoustique étant configuré pour identifier et enregistrer les signaux acoustiques de fond.

14. Dispositif de détection acoustique portable selon la revendication 13, comprenant en outre un processeur destiné à supprimer les signaux acoustiques de fond et isoler le signal acoustique cible.
